# EUROPEAN PATENT SPECIFICATION

(11) **EP 1 270 022 B1**
(45) Date of publication and mention of the grant of the patent: **15.09.2004**
(21) Application number: 02013690.9
(22) Date of filing: 20.06.2002
(51) Int. Cl.: A61L 9/03, A01M 1/20

(54) **Electrical vapour diffuser equipment**
Elektrisches Dampfdiffusionsbetriebsmittel
Appareillage électrique de diffusion de vapeur

(30) Priority: 21.06.2001 ES 200101442
(43) Date of publication of application: 02.01.2003
(73) Proprietor: BLAU BARCELONESA D'ACTIVITATS COMERCIALS, S.A., 08820 El Prat de Llobregat (Barcelona) (ES)
(72) Inventor: Boadella I Esteve, Oriol, 08820 El Prat de Llobregat(Barcelona) (ES)
(74) Representative: Maldonado Jordan, Julia

(56) References cited:
- EP-A- 1 175 833
- WO-A-98/19526
- WO-A-98/58692
- GB-A- 2 357 035

## Description

### AIM OF THE INVENTION

This present invention refers, as its title would indicate, to electrical vapour diffuser equipment. It deals with a device that is connected to the electricity supply and is intended to spread a vaporised product into the air that can be perfumed, deodorant or an insecticide.

### BACKGROUND TO THE INVENTION

At the present time, there are several pieces of equipment designed to spread vapours into the air and which work by the vaporising of a product that is initially in a liquid state.

Several types of electrical equipment evaporating liquids are known for the perfuming and/or repelling of insects, see e.g. GB-A-2 357 035. All of these have features that are not to be found in the equipment object of this present patent, in regard to which they cannot be considered in themselves as coming from them or prejudicing their novelty.

EP 0736248 (FALP) describes a piece of evaporator equipment that lacks an intensity flow regulator for the vapours produced (the present invention has two devices for the regulation, one mechanical and one electronic). The holding of the container is made by means of some fingers or appendixes (18) that affect an outlet (17) of the neck of the container.

WO 98/58692 (DBK) lacks a security thread and involves a double screw on the neck of the container, with a wide helicoidal groove or slot (8) and a wide space to the edge of the thread (11), which has a narrow passage. Into said groove or slot, the ends (9) of some fingers or stems (10) are introduced to control the vertical movement of the container. The regulation is carried out by the simple turning of the container in the body of the equipment, by sliding the wide worm (89) against the fingers (10). The regulation is not possible if this type of special container is not used, with a double thread.

WO 98/19526 (Zobele) has a piece of equipment without the safety thread (the piece of equipment in the invention has it in all of its versions). It also has a device for the regulation that is made up of an arm that turns at an angle, with an end that exits to the outside and which is moved by the user to produce the raising or lowering of the container. The screwing of the container means the changing of the position in respect of the intensity regulator, something that does not happen in the equipment in the invention.

### DESCRIPTION OF THE INVENTION

The electrical vapour diffuser has the following features according to claim 1:
a) A safety device that prevents child from meddling with the equipment and, specifically separating the container from the liquid to be vaporised and gaining access to same;
b) A mechanical intensity regulator for the product to be vaporised and spread, in which the capillary diffuser wick, fitted to a liquid product container is moved against an heating component that remains static against the body of the equipment;
c) Some stops in the mechanical regulator, determining the maximum, intermediate and minimum positions for the flow of the evaporated product;
d) An electronic regulator that acts on the electrical supply for the heater element;
e) A visual system indicator, fitted to the mechanical regulator, for the degree of movement of this to vary the flow of the evaporated product.

The safety device a) and the mechanical regulator b) for the flow of the evaporated product are structurally linked.

Preferred embodiments of the present invention are set out in the dependent claims.

In order to make the explanation easier, the present description is accompanied by some figures in which a presentation for the carrying out of the vapour diffuser equipment is shown in accordance with the principles of the claims. This is by way of description and is without limitation.

### DESCRIPTION OF THE FIGURES

- Figure 1 shows a lateral view of the new vapour diffuser, partially in section.
- Figures 2 and 3 show respective dihedral views of a first element that forms part of the equipment safety device.
- Figures 4 to 7 show respective dihedral partially sectioned views of a second element that form part of the equipment safety device.
- Figures 8 to 10 show respective dihedral partially sectioned views of a third element that form part of the equipment safety device.
- Figures 11 and 12 show the movement of the diffuser wick against the heater element, in its two extreme positions.
- Figures 13 and 14 show the shells that make up the electrical heater element of the capillary diffuser wick for the product.
- Figure 15 show the two heater element contact pieces and their connections, and figures 16 and 17 show the heater externally and the guide for the capillary wick diffuser.
- Figure 18 shows the electronic regulator for the supply to the heater element.
- Figure 19 shows the external aspect of the new diffuser equipment, in one of its possible presentations.
- Figures 20, 21 and 22 represent respective dihedral views of the hemispherical pieces that make up the equipment casing in accordance with figure 1, and figure 23 is a section of the piece itself in a plan view shown by XXIII-XXIII in figure 20.
- Figures 24 and 25 show presentations of the respective external shapes of the previous one.

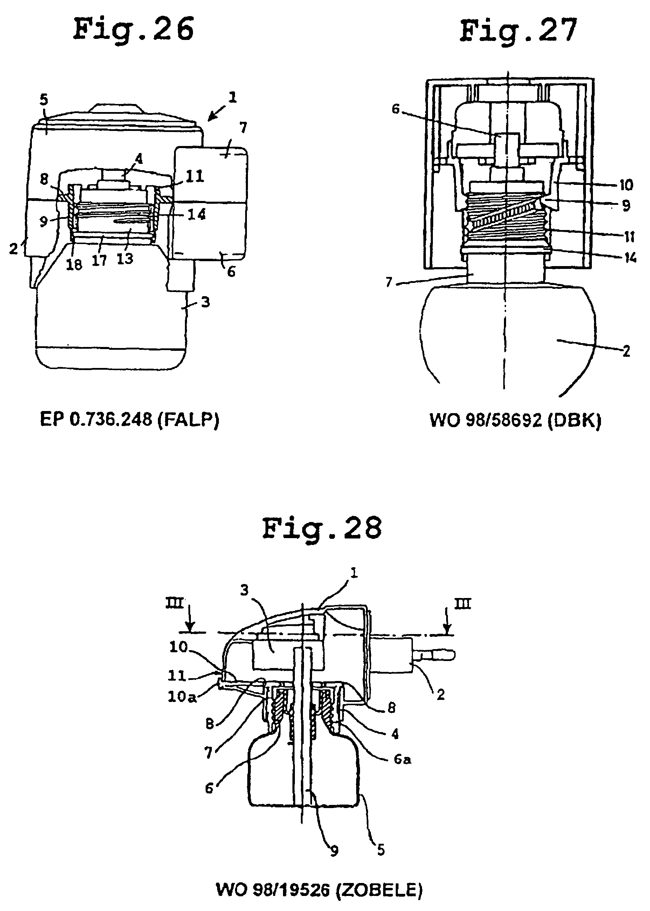

- Figures 26 to 28 show respective versions of volatile liquid evaporator equipment, corresponding to the known background in the previous state of the technique and in respect of which the equipment of this invention shows features of novelty.

### PREFERRED PRESENTATION OF THE INVENTION

The elements represented by numbers in the figures correspond to the parts stated below.

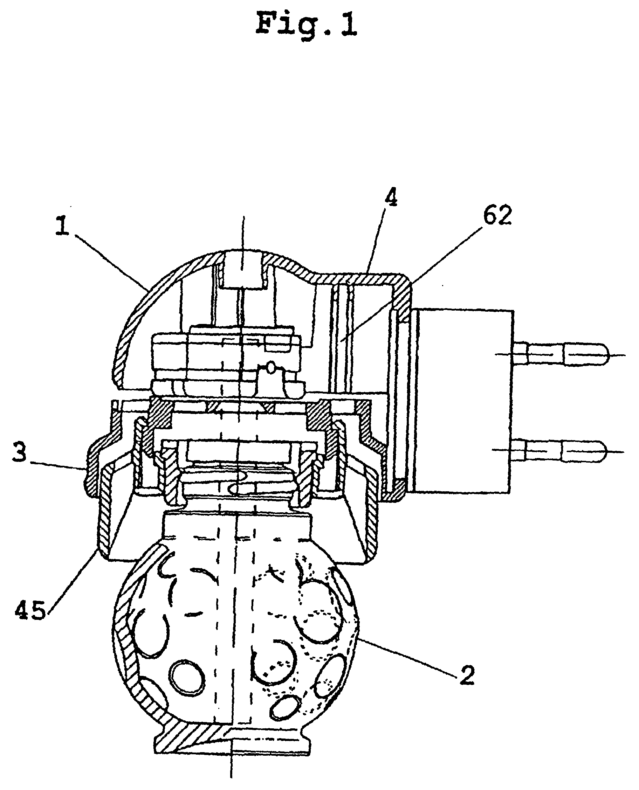

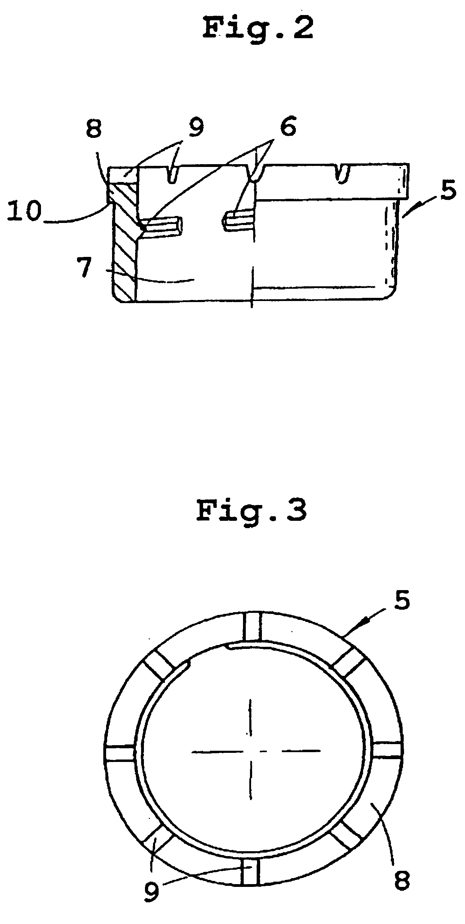

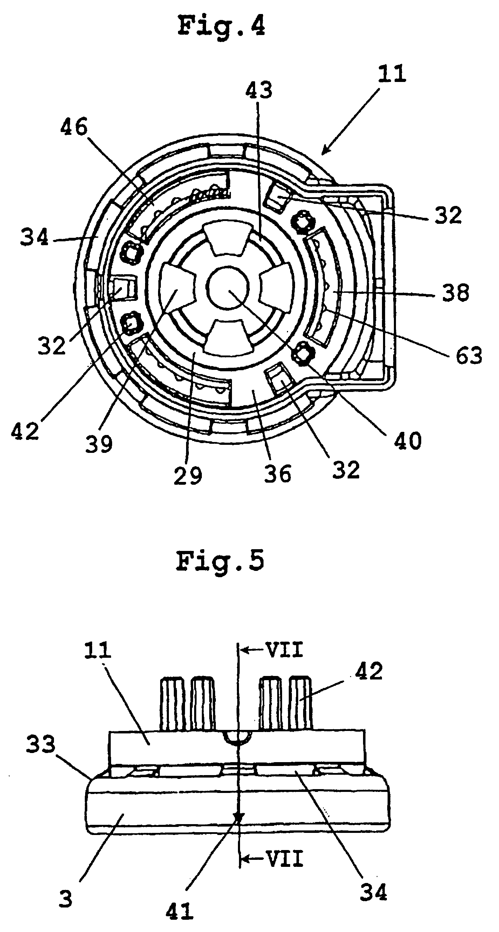

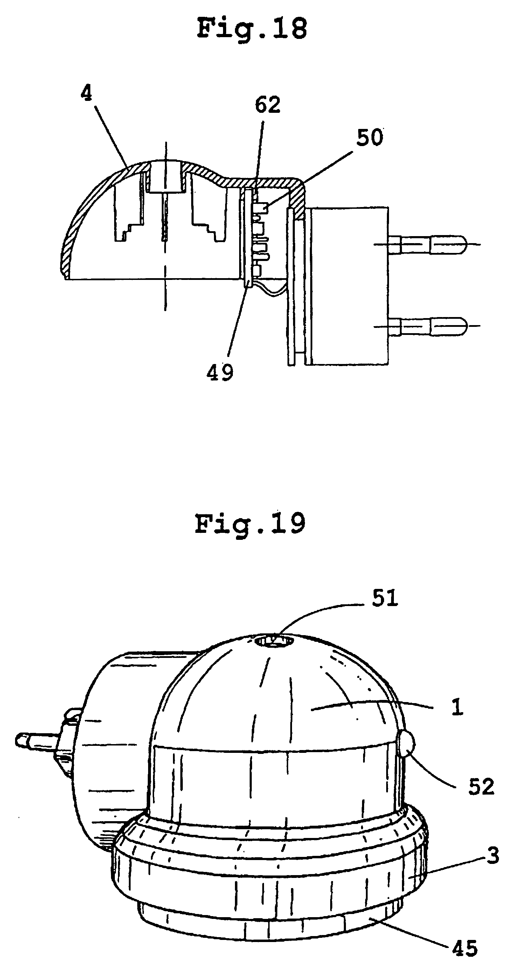

The vapour diffuser device object of this patent is basically made up of a body formed by a casing/carcass in which the following are housed: the heating element, the safety device, part of a mechanical regulator mechanism (which has another part protruding), an electronic regulator for the supply to the equipment and the mouth for the container that holds the liquid product destined to be vaporised and the porous wick and diffuser.
a) The casing/carcass (1) externally has, in the preferred version in accordance with figure 1, an incomplete spherical shape that can be seen in figure 19, coming from a cylindrical mouth (3) for the mechanical regulator, the safety device and the container mouth (2). Likewise, the casing/carcass (1) in the expansion being almost cylindrical (4) which will make contact pins to electricity supply wall socket.
b) The safety device guarantees that, once the container (2) is connected to the body of the device by means of an express and complicated movement that will be described below, the separation of the container is not feasible without carrying out another complicated express movement.
   Making up the safety device:
   - The element (5) figures 2 and 3 with a cylindrical-angular shape, with the helicoidal screw slot (6) on the inner wall (7), the rim (8), the upper notches (9) and the external step (10);
   - The element (11), figures 4 to 7, in the general shape of a plate, completed by the hemispherical part (1) the casing/carcass and involves the following parts:

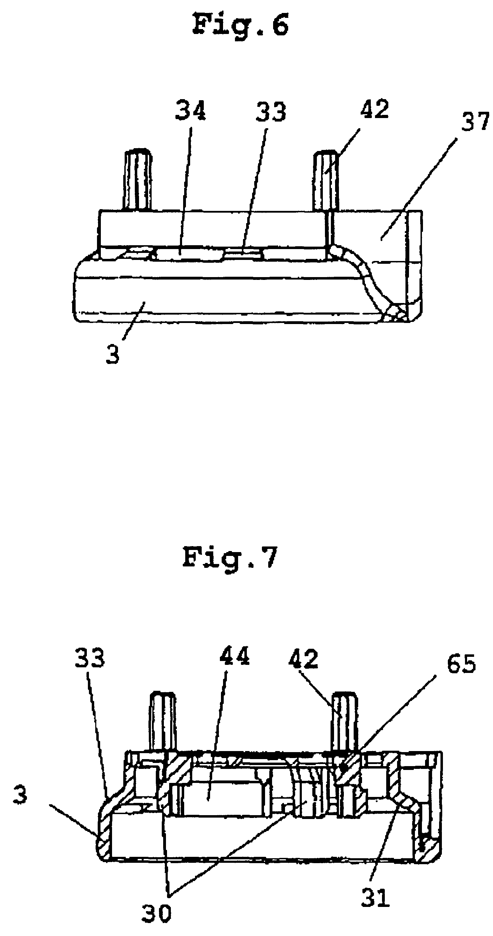

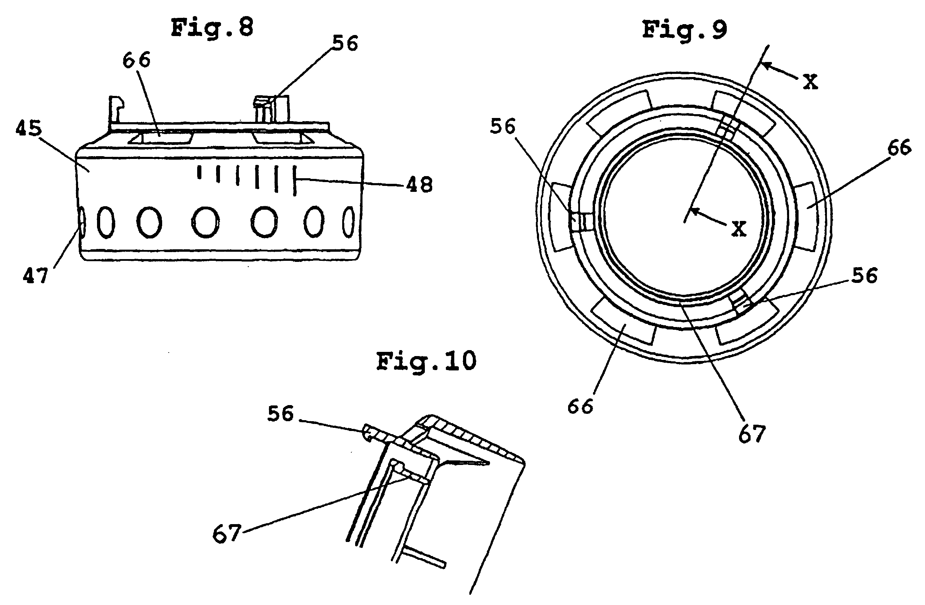

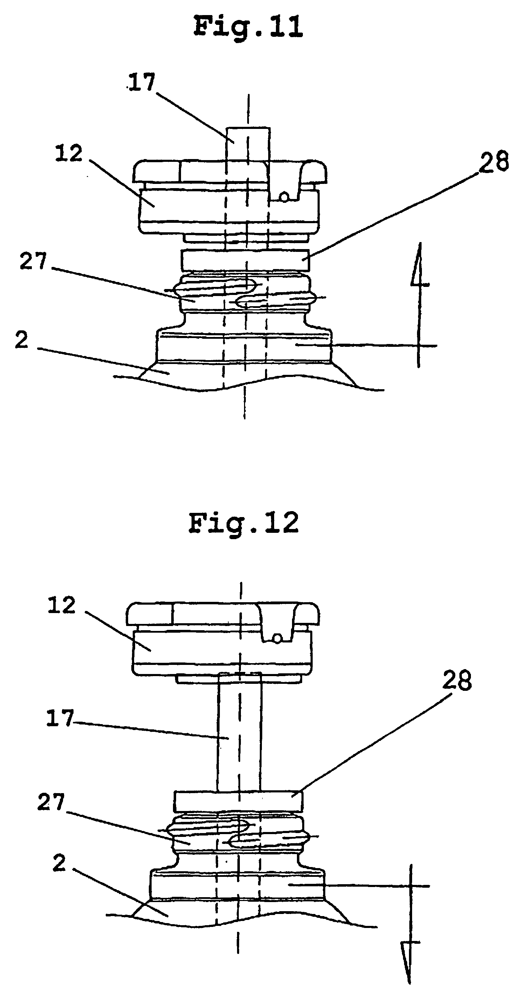

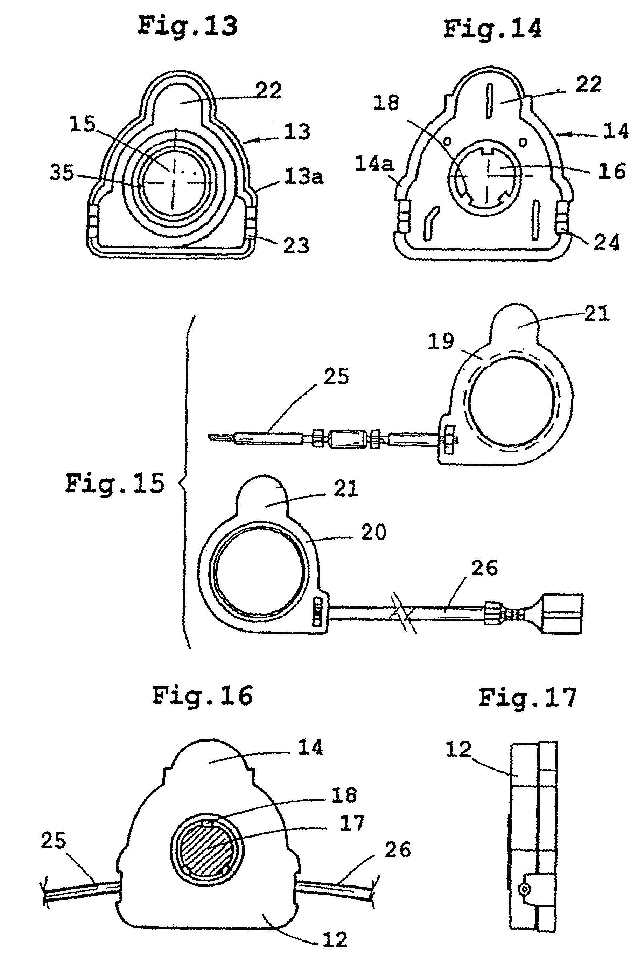

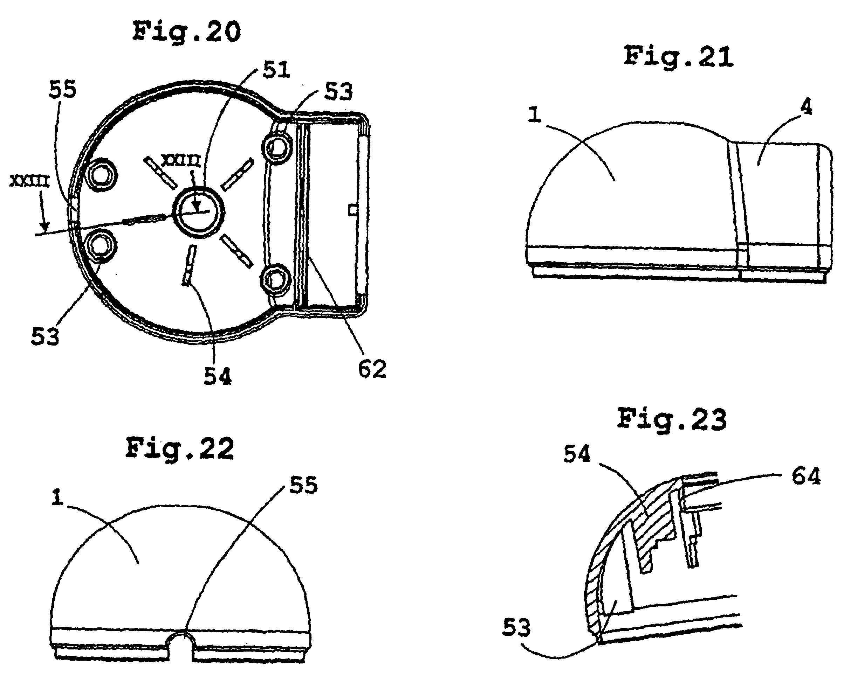

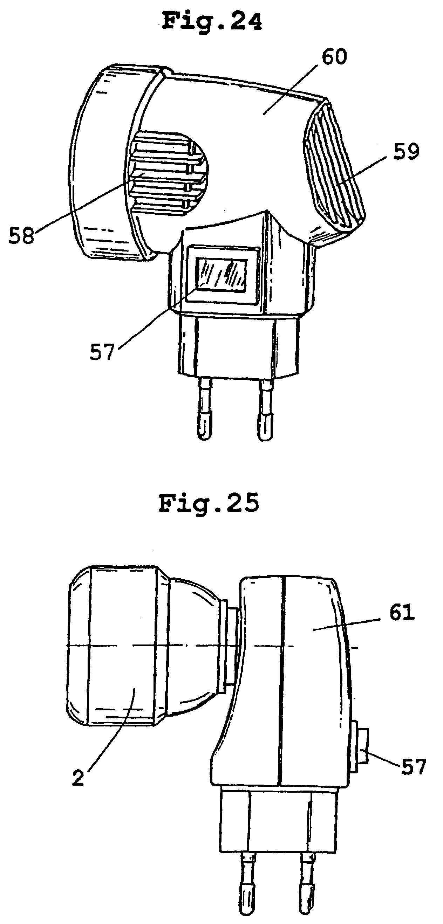

   The cylindrical flap (3), the centre ring (29) (the lower face which has lugs (65), seen in figure 7, in circular order and combined with the notches (9) of element (5)), the rectilinear protrusions and cuts (30) on the lower face, the side area (31) of the flap (3), the openings (32) to make the removal from the mould easier, the peripheral oblique area (33) with the openings (34), which are used for the entry and circulation of air into the inside of the equipment, to the exit chimney (64) for the flow of the evaporated product, visible in figure 23, the ring (36), the lateral expansion area (37) joined to the upper area (4) of the casing/carcass figures 6 and 7, the curved line openings (38) to make the removal from the mould easier, the trapezoidal openings (39), with the same function as (34), the central opening (40) to allow for the passage of the wick (17), the stems (42) that are used for the coupling of the element (11) with the upper hemispherical part (1), the central ribs in an arc (43), which are used for the positioning of the heating element (12), the curved line protrusion guides (44) with a height constant with that of the lower face, provided with lugs or protrusions (63), and the curved line protrusions (46) having a decreasing height, which are used for the angular turn and the simultaneous rectilinear advance of the flow regulator device of the evaporated product.
   The cylindrical element (45), figures 8 to 10, has at the upper end forming openings (66) distributed in an arc in correspondence with openings (34), these being used for the circulation of the air on the inside of the equipment; having on the same upper part some appendixes (56) for the coupling to the element (11).
   The flap (45) on the external surface joins some protrusions (47) designed to make the activating of the element easier in its rotation, likewise some marks (48) (for example, in the shape of longitudinal decreasing parallel lines), indicating the degree of regulation of the device. Such marks are combined with an arrow for reference (41) at the area (3) of the element (11). The ring (67) is used for the housing of the element (5) that can rotate freely.
c) The container (2), figures 11 and 12, has an external threaded area (27) at its mouth and a support (28) for the cylindrical wick (17), and this occupies an axial position against the previous elements.
d) The heater element, shown by way of example in figures 13 to 17, is made up of a box (12) in general with a flattened triangular prismatic shape, made up of two semi-boxes (13 and 14), with respective central openings (15 and 16) for the passage of the wick (17), being fitted with at least one of the internal lugs (18) which act as guides for the centring of the wick, and the other opening of the neck (35).
   The angular metal pieces (19 and 20) are housed in the semi-boxes (13 and 14), making the contacts for the heating element, and between the expansions (21) there is an electro-ceramic block made from a PTC type of material (positive temperature coefficient), which heats up when the current is passed. In the openings (15, 16) of the semi-boxes (13 and 14) the neck (35) is shown respectively and the already mentioned centring lugs (18). The appendixes (21) of the contact pieces are in turn housed in the expansions (22) of the semi-boxes (13, 14), and in the areas (13a and 14a) of these forming notches (23, 24) for the passage of the electrical contacts (25, 26), entering on opposing sides of the box (12) of the heater element.
f) The electronic regulator, figure 18, is made up of a printed circuit board (49), carrying components (50) which comprises of a timer and activator assembly for the heater electricity supply (12). The board is held between two parallel ribs (62) on the internal side of the piece (4).
g) The opening (51) figure 19 at the top of the hemispherical part (1) allows the escape of the vapours caused by the heating of the capillary wick and its release into the surrounding space.
h) The pilot light (52) on the front part of the body of the equipment shows the state of connection to the current and the working of the vapour diffuser.
i) The hemispherical body (1) of the casing/carcass has female columns (53) on the inside figures 20 to 23 in order to ensure the fitting together of the body with the stems (42) of the heater element (12) (the section can be seen in figure 23), the slot (55) for the pilot light (52), and the cylinder or chimney (64) that shapes the opening (51) of the outlet for the vapours to be released.
j) The versions in figures 24 and 25, with a different geometric exterior, have a switch on the side (57), side openings (58) for the entry of air and openings (59) at the end for the exit of the vapours generated, placed in the casing/carcass (60 and 61), respectively.

With the elements stated, and with reference to figure 1, the container (2) is fitted into the casing/carcass (1) by the insertion of the wick (17) into the central opening (40) of the element (11) and into the central opening of the heater element (12), and by the introduction of the threaded neck (27) of the container into the cylindrical ring shaped element (5), where the helicoidal screw (6) holds said neck (27).

The element (5), coaxial with element (11) and with the ring (67) shaped by element (45), is initially detached from both and, hence, can turn freely in them.

Thus, the container is free to turn in the casing/carcass (1).

For the effective holding of the container (2), force is applied with a certain degree of intensity in an axial direction and in an upward direction as shown in figure 1, with which the notches (9) of the turning element (5) correspond with the lugs (65) placed on the inner face of the fixed element (11), this (5) becoming momentarily fixed, which allows the screwing of the neck (27) of the container and the effective fixing of same in place, being solidly fixed with the elements (11 and 45).

The container (2) and the element (5) can now be turned together, being separated (by gravity) from the fixed element (11) due to the exit (separation) of the notches (9) from the element (5) against the lugs (65) of it.

But the container and the element (5) cannot be separated from the casing/carcass (1) as a result of the element (5) being held by the periphery (step 10).

When the effective separation of the container (2) is desired, for example after the liquid contained is used up, once again force is applied in an upward axial direction, with which the element (5) is momentarily immobilised in (11) by meeting with the notches (9) and the lugs (65) and the neck (27) can be unscrewed from it (5).

The safety of the equipment in regard to the possibility of it being meddled with by a child lies in the fact that, normally, he/she will not be able to apply the axial force and the turn necessary on his/her own to be able to separate the neck (27) from its housing, and will only be able, in such situation, to turn the container around on its axis.

The mechanical graduation of the vapour flow expelled by the equipment is the result of the sliding of the fingers (56) of the rotating element (45) along the protruding curved lines (46) having decreasing heights from the fixed element (11). This varies the axial position of (45) and thus, the element (5) held by the external step (10) on the ring (67), the container (2) being more or less introduced into the opening (27) and consequently the wick (17) in the heater (12) as shown in figures 11 and 12.

The lugs (63) of the protrusions (46) determine differing positions for the contact of the fingers or appendixes (56) of the revolving element (45), therefore, differing degrees of the introduction of the wick (17) in the heater (12).

## Claims

1. Electrical equipment for vapour diffusion, being of the type made up of a container (2) holding a product in a liquid state to be evaporated and a diffuser wick (17), movable against an electrical heating element (12) fixed into the casing/carcass (1) of the device, a safety device fitted to a mechanical intensity regulator (45) for the flow of the evaporated product, visual indicator (48) device for the said intensity of the flow and an electronic regulator for the supply to the heater, **characterised in that** the safety device is functionally coupled to the mechanical regulator device (45) and is made up from:
a) A tubular cylindrical element (5) with internal thread,
b) A cylindrical-annular element (11) fitted with openings (34) and protrusions (30) surrounding the previous element, and
c) A disk shaped element (36) fitted with openings (38, 39, 40) and protrusions (44, 46 and 65) defining in its entirety an operative group that allows for the initial coupling of the container (2) and the wick (17) by means of force in an upward axial direction, the thread of the neck (27) of the container (2) and the retention of the latter with the possibility of free rotational movement in both directions, requiring another upward force to achieve the possibility of separation of the container by unscrewing.

2. Electrical equipment for vapour diffusion, in accordance with claim 1, is **characterised in that** the component (5) of the safety device is made up with the screw thread (6) on its internal face for the attachment of the container (2) of the product to be evaporated, a rim (8) with multiple upper notches (9) and an exterior step (10).

3. Electrical equipment for vapour diffusion, in accordance with claim 1, is **characterised in that** the component (11) of the safety device, in the general shape of a plate, is made up of an upper groove (29), three vertical fingers (30), a lower ring (31) with openings (32), an external oblique area (33) with openings (34) and the cylindrical flap (3) which partially protrudes from the casing/carcass (1).

4. Electrical equipment for vapour diffusion, in accordance with claim 1, is **characterised in that** the element (11) of the safety device has a lateral expansion (37) on its circular (36) part corresponding to the expansion (4) of the case/carcass (1), and has the openings (38) on its upper surface in an arch, the openings (3) in a trapezoidal shape and the central circular opening (40), likewise on its lower side, the protrusions (65) fitted together with the notches (9) of the element (5).

5. Electrical equipment for vapour diffusion, in accordance with claim 1, is **characterised in that** the cylindrical flap (45) of the mechanical regulator device for the intensity flow of the evaporated product, turns on an axis that is in line with the body (1) of the equipment and emerging in respect of same by a length proportional to the rate of the exit flow of the evaporated product, having external means (48), such as lines of differing lengths, marking out the amount of said rate of flow.

6. Electrical equipment for vapour diffusion, in accordance with claim 1, is **characterised in that** the heater device remains housed and fixed in the internal upper part of the casing/carcass (1) by several positioning guides (54).

7. Electrical equipment for vapour diffusion, in accordance with claim 1, is **characterised in that** on the front part of the casing/carcass (1) there is a luminous indicator (52) showing the working state of the device, provided by a light emitting diode (LED).

8. Electrical equipment for vapour diffusion, in accordance with claim 1, is **characterised in that** on the upper part of the casing/carcass (1) there is an opening (51) for the exit of the vaporised product, connected to a chimney tube (64) on the inside.

## Patentansprüche

1. Elektrischer Dampfzerstäuber bestehend aus einem Behälter (2), der ein Produkt in flüssigem Zustand enthält, welches verdampft werden soll, einer gegenüber einem am Hüllkörper (1) der Vorrichtung fixierten elektrischen Heizelement (12) verschiebbaren Zerstäuberkapillare (17), einer an einen mechanischen Intensitätsregler (45) zur Regulierung des ausströmenden Produktdampfes gekoppelten Sicherheitsvorrichtung, einer visuellen Anzeige (48) für diese Strömungsstärke und einem elektronischen Regler zur Versorgung der Heizung, **dadurch gekennzeichnet, dass** die Sicherheitsvorrichtung funktionell an eine mechanische Regelvorrichtung (45) gekoppelt ist und aus
a) einem zylindrischen Rohrelement (5) mit Innengewinde,
b) einem zylindrischen Ringelement (11) mit Ausnehmungen (34) und Vorsprüngen (30), die rund um dieses Element angeordnet sind, und
c) einem scheibenförmigen Element (36) mit Ausnehmungen (38, 39, 40) und Vorsprüngen (44, 46, 45), welche zusammen eine operative Einheit bilden, die eine erste Ankopplung des Behälters (2) an das Kapillarelement (17) durch eine in Axialrichtung nach oben ausgeübte Kraft, das Eindrehen des Halses (27) des Behälters (2) und die Fixierung desselben bei gleichzeitiger freier Drehbarkeit in beide Richtungen ermöglicht, wobei eine weitere Krafteinwirkung nach oben nötig ist, um den Behälter durch losdrehen wieder vom Kapillarelement trennen zu können.

2. Elektrischer Dampfzerstäuber nach Anspruch 1, **dadurch gekennzeichnet, dass** das Bauteil (5) der Sicherheitsvorrichtung auf seiner Innenseite ein Gewinde (6) zur Befestigung des Behälters (2), der das zu verdampfende Produkt enthält, einen vorstehenden Rand (8) mit zahlreichen oberen Einschnitten (9) und einen äußeren stufenförmigen Absatz (10) aufweist.

3. Elektrischer Dampfzerstäuber nach Anspruch 1, **dadurch gekennzeichnet, dass** das Bauteil (11) der Sicherheitsvorrichtung, welche generell eine abgeflachte Form hat, eine obere Rille (29), drei vertikale Finger (30), einen unteren Ring (31) mit Ausnehmungen (32), einen schrägen äußeren Bereich (33) mit Ausnehmungen (34) und einen zylindrischen Flügel (3) der zum Teil über den Hüllkörper (1) hinausragt, aufweist.

4. Elektrischer Dampfzerstäuber, nach Anspruch 1, **dadurch gekennzeichnet, dass** das Bauteil (11) der Sicherheitsvorrichtung in seinem kreisförmigen Teil (36) eine der Ausweitung (4) des Hüllkörpers (1) entsprechende seitliche Ausweitung (37) aufweist, sowie auf seiner Oberseite bogenförmige Ausnehmungen (38), trapezförmige Ausnehmungen (3) und die kreisrunde Öffnung (40) in der Mitte, ebenfalls auf seiner Unterseite, wobei die Erhebungen (65) in die Einschnitte (9) des Bauteils (5) eingreifen.

5. Elektrischer Dampfzerstäuber, nach Anspruch 1, **dadurch gekennzeichnet, dass** sich der zylindrische Flügel (45) der mechanischen Regelvorrichtung zur Regulierung der Strömungsstärke des Produktdampfes um eine mit dem Hüllkörper (1) des Dampfzerstäubers gleichgerichtete Achse dreht, welche über diesen um eine Länge hinausragt, die proportional zur Austrittsmenge des Dampfproduktes ist, wobei besagter Flügel äußere Mittel (48), wie etwa Linien unterschiedlicher Länge, die die Austrittsmenge kennzeichnen, aufweist.

6. Elektrischer Dampfzerstäuber, nach Anspruch 1, **dadurch gekennzeichnet, dass** das Heizelement im inneren oberen Teil des Hüllkörpers (1) untergebracht und durch mehrere Halterungen (54) fixiert ist.

7. Elektrischer Dampfzerstäuber, nach Anspruch 1, **dadurch gekennzeichnet, dass** an der Vorderseite des Hüllkörpers (1) eine mit einer Leuchtdiode (LED) ausgestattete Leuchtanzeige (52) angebracht ist, welche die Betriebssituation der Vorrichtung anzeigt.

8. Elektrischer Dampfzerstäuber, nach Anspruch 1, **dadurch gekennzeichnet, dass** der Hüllkörper (1) auf seiner Oberseite eine Öffnung (51) für den Austritt des Produktdampfes aufweist, an die im Inneren ein Abzugsrohr (64) angeschlossen ist.

## Revendications

1. Diffuseur de vapeur électrique du type constitué par un récipient (2) qui contient un produit en état liquide qui doit être vaporisé et un élément diffuseur par capillarité (17) qui peut être déplacé par rapport à un élément de chauffage électrique (12) fixé au corps (1) du dispositif, un dispositif de sécurité accouplé à un régulateur d'intensité de type mécanique (45) pour réguler le flux de produit vaporisé, un indicateur visuel (48) de ladite intensité de flux et un régulateur électronique pour l'alimentation du dispositif de chauffage, **caractérisé en ce que** le dispositif de sécurité est accouplé fonctionellement au dispositif de régulation mécanique (45), étant constitué par:
a) un élément cylindrique tubulaire (5) fileté à l'intérieur,
b) un élément cylindrique annulaire (11), présentant des ouvertures (34) et des éléments faisant saillie (30) qui entourent cet élément, et
c) un élément (36) en forme de disque, présentant des ouvertures (38,39,40) et des éléments faisant saillie (44,46,45) qui définent dans leur ensemble un groupe fonctionnel qui permet l'accouplement initial du récipient (2) à l'élément de capillarité (17) moyennant une force exercée en direction axiale vers de haut, le vissage du goulot (27) du récipient (2) et la retenue de ce dernier, permettant un mouvement de libre rotation dans les deux sens, et étant nécessaire une autre force vers le haut pour rendre posible la séparation du récipient par dévissement.

2. Diffuseur de vapeur électrique selon la revendication 1, **caractérisé en ce que** le composant (5) du dispositif de sécurité présente à son côté intérieur un filetage (6) destiné à y accoupler le récipient (2) qui contient le produit vaporiser, un rebord (8) avec de multiples rainures supérieures (9) et un échelon extérieur (10).

3. Diffuseur de vapeur électrique selon la revendication 1, **caractérisé en ce que** le composant (11) du dispositif de sécurité, qui a généralement une forme aplatie, présente une rainure supérieure (29), trois doigts verticaux (30), un anneau inférieur (31) avec des ouvertures (32), une zone oblique externe (33) avec des ouvertures (34) et une aile cylindrique (3) parciellement en saillie par rapport au corps (1).

4. Diffuseur de vapeur électrique selon la revendication 1, **caractérisé en ce que** l'élément (11) du dispositif de sécurité présente une expansion latéral (37) dans sa partie circulaire (36), qui correspond à l'expansion (4) du corps (1), et les ouvertures (38) sur son côté supérieur en forme d'arc, les ouvertures (3) trapézoïdales et l' ouverture circulaire central (40), également sur son côté inférieur, étant les éléments faisant saillie (65) accouplées aux les rainures (9) de l'élément (5).

5. Diffuseur de vapeur électrique selon la revendication 1, **caractérisé en ce que** l'aile cylindrique (45) du dispositif de régulation mécanique, qui sert à réguler l'intensité du flux du produit vaporisé, tourne sur un axe aligné avec le corps (1) de l'appareil et qui est en saillie par rapport à ce dernier dans une longueur qui est proportionnelle à la quantité de produit vaporisé, étant muni ladite aile de moyens externes (48) tels que des lignes de différentes longueurs qui marquent la magnitude du débit de produit.

6. Diffuseur de vapeur électrique selon la revendication 1, **caractérisé en ce que** le dispositif de chauffage reste logé et fixé dans la partie supérieure interne du corps (1) à l'aide de plusieurs guides de positionnement (54).

7. Diffuseur de vapeur électrique selon la revendication 1, **caractérisé en ce qu'** au côté frontal du corps (1) se trouve un indicateur lumineux (52), qui montre la situation de fonctionnement du dispositif, et qui est équipé d'une diode électroluminescente (DEL).

8. Diffuseur de vapeur électrique selon la revendication 1, **caractérisé en ce qu'** à la part supérieure de corps (1) se trouve un orifice (51) permettant la sortie du produit vaporisé et qui est conecté à un tuyau cheminée (64) à l'intérieur.
